(19) Europäisches Patentamt
European Patent Office
Office européen des brevets

(11) **EP 3 462 381 A1**

(12) **DEMANDE DE BREVET EUROPEEN**

(43) Date de publication:
03.04.2019 Bulletin 2019/14

(51) Int Cl.:
*G06K 9/00* (2006.01)          *C12Q 1/04* (2006.01)
*G01N 33/02* (2006.01)         *G01N 15/14* (2006.01)
*G03H 1/04* (2006.01)

(21) Numéro de dépôt: 18196638.3

(22) Date de dépôt: 25.09.2018

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR
Etats d'extension désignés:
BA ME
Etats de validation désignés:
KH MA MD TN

(30) Priorité: 27.09.2017 FR 1758973

(71) Demandeur: Commissariat à l'Énergie Atomique
et aux Énergies Alternatives
75015 Paris (FR)

(72) Inventeurs:
• BORDY, Thomas
  38054 Grenoble cedex 09 (FR)
• CIONI, Olivier
  38054 Grenoble cedex 09 (FR)
• DEFORCEVILLE, Camille
  46120 ANGLARS (FR)

(74) Mandataire: GIE Innovation Competence Group
310, avenue Berthelot
69372 Lyon Cedex 08 (FR)

(54) **PROCÉDÉ DE DÉTECTION DE MICROORGANISMES DANS UN ÉCHANTILLON**

(57) L'invention est un procédé de détection de microorganismes ($10_m$) dans un échantillon, l'échantillon comportant également des particules dites alimentaires ($10_o$), formées à partir d'aliments, le procédé comportant l'illumination de l'échantillon et l'acquisition d'une image de ce dernier par un capteur d'image, le capteur d'image étant alors exposé à une onde lumineuse dite d'exposition. Le procédé comporte les étapes suivantes :
▪ détermination de positions ($x_i, y_i$) de particules d'intérêt ($10_i$) susceptibles de correspondre à un microorganisme ;
▪ à partir de l'image acquise ($I_0$) par le capteur d'image, application d'un opérateur de propagation ($h$), de façon à calculer au moins une grandeur caractéristique ($M, \varphi$) de l'onde lumineuse d'exposition (14), à chaque position radiale ($x_i, y_i$), et à une pluralité de distances ($z$) du plan de détection ($P_0$);
▪ formation d'un profil ($M_i(z), \varphi_i(z)$), représentant une évolution de la grandeur caractéristique entre le capteur d'image et l'échantillon ;
▪ identification des microorganismes ($10_m$) en fonction de chaque profil

$10_i$

**Fig. 3E**

EP 3 462 381 A1

## Description

## DOMAINE TECHNIQUE

Le domaine technique de l'invention est la détection de microorganismes, notamment des levures, dans une matrice, en particulier une matrice solide ou liquide.

## ART ANTERIEUR

L'utilisation de microorganismes tels des levures ou des bactéries, ou leurs dérivés, trouve de nombreuses applications dans de nombreux domaines. Dans le domaine de l'agroalimentaire, par exemple, l'utilisation de levures est répandue dans différents secteurs, tels la boulangerie, la production de vin, la brasserie ou encore la fabrication de produits laitiers. L'application de levures ou de bactéries concerne de nombreux aliments par le biais de probiotiques, ces derniers étant par exemple ajoutés à des céréales ou à des aliments pour animaux. En dehors de l'agroalimentaire, de nombreux domaines industriels peuvent mettre en oeuvre des microorganismes. Il s'agit par exemple de l'agriculture ou de l'horticulture, avec le développement de produits phytosanitaires ou de fertilisants plus respectueux de l'environnement, ou encore de la production de biocombustibles obtenus à partir de végétaux. D'autres applications concernent le domaine de la pharmacie et du diagnostic médical.

Lorsque des levures sont incorporées dans un produit, par exemple un produit alimentaire, il peut être nécessaire de vérifier la quantité des levures ajoutées, à des fins de contrôle qualité. Les contrôles peuvent par exemple être élaborés au microscope, sur des échantillons. Il s'agit d'un contrôle fastidieux, effectué visuellement par un opérateur. Il est difficilement automatisable. De plus, le champ d'observation d'un microscope étant réduit, il est nécessaire de déplacer relativement l'échantillon par rapport au microscope de façon examiner une surface significative de l'échantillon.

Par ailleurs, le document WO2016151248 décrit un procédé permettant l'identification de particules, les particules étant des cellules ou des microorganismes, par exemple des levures. Dans ce document, les particules sont réparties dans un liquide, en particulier du sang. L'identification est réalisée à partir d'une image acquise selon une configuration d'imagerie sans lentille. Plus précisément, l'identification est obtenue à partir de profils établis à partir d'images obtenues par reconstruction holographique à partir de l'image acquise.

Le document WO2016/118884 décrit un procédé d'observation et de comptage de particules selon une configuration d'imagerie sans lentille. Le procédé comporte l'application d'un film fin sur les particules, de façon à former une microlentille à la surface de particules, selon des principes décrits dans EP2872870.

Le document WO2016097092 décrit un procédé permettant une identification de bactéries à partir d'une image acquise selon une configuration défocalisée.

Les inventeurs ont proposé une alternative à la méthode optique tradtionnelle, de façon à effectuer détection automatisable de microorganismes, telles des levures, dans un échantillon complexe. L'objectif est par exemple d'obtenir une détermination rapide et automatisable du nombre, ou de la concentration, de levures présentes dans cet échantillon.

## EXPOSE DE L'INVENTION

Un premier objet de l'invention est un procédé de détection de microorganismes dans un échantillon, l'échantillon comportant également des particules dites alimentaires, formées à partir d'aliments, le procédé comportant les étapes suivantes :

    a) illumination de l'échantillon à l'aide d'une source de lumière, la source de lumière émettant une onde lumineuse incidente se propageant vers l'échantillon selon un axe de propagation;
    b) acquisition, à l'aide d'un capteur d'image, d'une image de l'échantillon, formée dans un plan de détection, l'échantillon étant disposé entre la source de lumière et le capteur d'image, l'image acquise étant représentative d'une onde lumineuse d'exposition, à laquelle est exposé le capteur d'image sous l'effet de l'illumination ;

le procédé étant caractérisé en ce qu'il comprend également les étapes suivantes :

    c) obtention d'une image d'observation de l'échantillon à partir de l'image acquise lors de l'étape b) et sélection, dans l'image d'observation, de régions d'intérêt, chaque région d'intérêt correspondant à une particule d'intérêt, susceptible de correspondre à un microorganisme;
    d) détermination de positions radiales, dans un plan parallèle au plan de détection, des particules d'intérêt correspondant aux différentes régions d'intérêt sélectionnées;
    e) à partir de l'image acquise lors de l'étape b), application d'un opérateur de propagation, de façon à calculer au moins une grandeur caractéristique de l'onde lumineuse d'exposition, à chaque position radiale déterminée lors de l'étape d), et cela à une pluralité de distances du plan de détection;
    f) formation d'un profil, représentant une évolution de la grandeur caractéristique calculée lors de l'étape e) selon un axe parallèle à l'axe de propagation et passant par chaque position radiale déterminée lors de l'étape d), chaque profil étant associé à une particule d'intérêt;
    g) en fonction de chaque profil formé lors de l'étape f), identification des particules d'intérêt qui correspondent à un microorganisme parmi les particules d'intérêt sélectionnées lors de l'étape c).

L'étape c) peut comporter les sous-étapes suivantes :

ci) à partir de l'image acquise lors de l'étape b), application d'un opérateur de propagation, de façon à former une image complexe de l'échantillon, dans un plan de reconstruction dans lequel s'étend l'échantillon ;
cii) à partir de l'image complexe formée lors de la sous-étape ci), obtention de l'image d'observation de l'échantillon ;
ciii) application d'un critère à l'image d'observation, de façon à sélectionner les régions d'intérêt correspondant à des particules d'intérêt, représentatives d'un microorganisme.

Par image complexe de l'échantillon, on entend une image représentant une expression complexe représentative de l'onde lumineuse d'exposition dans le plan de reconstruction. L'image d'observation est par exemple obtenue à partir du module et/ou de la phase de l'image complexe de l'échantillon.
Lors de la sous-étape ciii), le critère appliqué peut être un critère morphologique, paramétré par une forme et/ou une dimension, de telle sorte que les régions d'intérêt sélectionnées sont celles s'étendant, dans l'image d'observation, selon ladite forme et/ou selon ladite dimension.
La sous-étape ciii) peut comporter, pour chaque région d'intérêt sélectionnée:

- une définition d'un seuil de niveau de bruit ;
- une définition d'un voisinage autour de la région d'intérêt ;
- une estimation d'un niveau de bruit dans le voisinage ;

de façon à retenir les régions d'intérêt sélectionnées dans le voisinage desquelles le niveau de bruit est inférieur au seuil de niveau de bruit, les autres régions d'intérêt étant rejetées.
La sous-étape ciii) peut comporter, pour chaque région d'intérêt sélectionnée :

- une définition d'une plage d'acceptation de niveaux d'intensité ;
- un calcul d'un niveau d'intensité représentatif de chaque région d'intérêt ;

de façon à retenir les régions d'intérêt sélectionnées pour lesquelles le niveau d'intensité est compris dans la plage d'acceptation, les autres régions d'intérêt étant rejetées.
L'étape g) peut comporter une détermination d'au moins un paramètre de chaque profil, l'identification des microorganismes étant effectuée en fonction dudit paramètre. Le paramètre est par exemple une valeur minimale du profil.
L'étape e) peut comporter une formation d'une pile d'images complexes, chaque image complexe étant représentative d'une expression complexe de l'onde lumineuse d'exposition, dans un plan de reconstruction. Les images complexes de la pile d'image complexes sont de préférence formées selon des plans de reconstruction parallèles les uns aux autres, et s'étendant entre le plan de détection et un plan de l'échantillon, selon lequel s'étend l'échantillon,et/ou de part et d'autre du plan de l'échantillon.
Le procédé peut comporter une détermination du plan de l'échantillon en mettant en oeuvre un algorithme de focalisation numérique.
Lors de l'étape f), la propriété optique peut être obtenue à partir du module ou de la phase d'une expression complexe représentative de l'onde lumineuse d'exposition.
Les microorganismes peuvent être des levures.
L'échantillon peut être obtenu par broyage d'un aliment comportant des microorganismes.
Le procédé peut comporter une étape h) de comptage des microorganismes identifiés lors de l'étape g).
Un autre objet de l'invention est un dispositif pour l'identification de microorganismes disposés dans un échantillon, le dispositif comportant:

- une source de lumière apte à émettre une onde lumineuse incidente se propageant vers l'échantillon;
- un capteur d'image ;
- un support, configuré pour maintenir l'échantillon entre la source de lumière et le capteur d'image ;
- un processeur, configuré pour recevoir une image de l'échantillon acquise par le capteur d'image et à mettre en oeuvre les étapes c) à g) d'un procédé selon le premier objet de l'invention.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

**FIGURES**

La figure 1 représente un exemple de dispositif selon l'invention.
La figure 2 illustre les principales étapes d'un procédé permettant d'identifier des microorganismes, dans un échantillon comportant des particules n'étant pas des microorganismes, par exemple des particules formées à partir d'aliments.
La figure 3A est une image au microscope d'un échantillon utilisé lors d'essais expérimentaux.

La figure 3B est une image d'observation de l'échantillon, représentant le module d'une image complexe. L'image complexe est obtenue par une reconstruction holographique à partir d'une image acquise selon une configuration sans lentille. L'image d'observation représentée sur la figure 3B montre une sélection de régions d'intérêt en fonction d'un premier critère morphologique.

La figure 3C est l'image d'observation de l'échantillon représentée sur la figure 3B, montrant une sélection de régions d'intérêt en fonction du premier critère morphologique ainsi que d'un deuxième critère, basé sur une estimation d'un niveau de bruit dans un voisinage défini autour de chaque région d'intérêt sélectionnée suite à l'application du premier critère morphologique.

La figure 3D est l'image d'observation de l'échantillon représentée sur la figure 3B, montrant une sélection de régions d'intérêt en fonction des premier et deuxième critères précédemment évoqués, ainsi que d'un troisième critère basé sur l'intensité des pixels dans chaque région d'intérêt sélectionnée suite à l'application du premier et du deuxième critères.

La figure 3E est une image d'observation de l'échantillon représentée sur la figure 3B, montrant une sélection de régions d'intérêt suite à l'application des premier, deuxième et troisième critères précédemment évoqués, ainsi que de l'analyse de profils tels que décrits en lien avec la figure 4A.

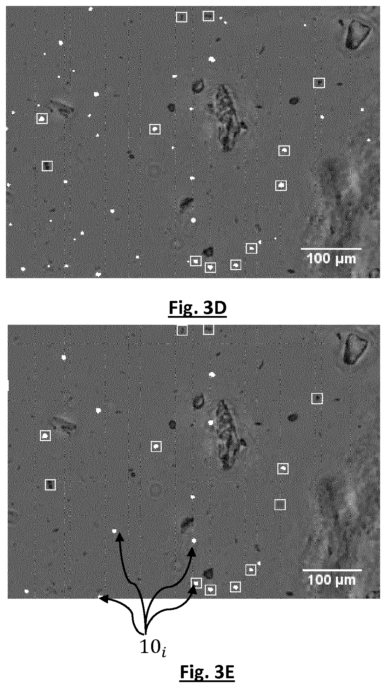

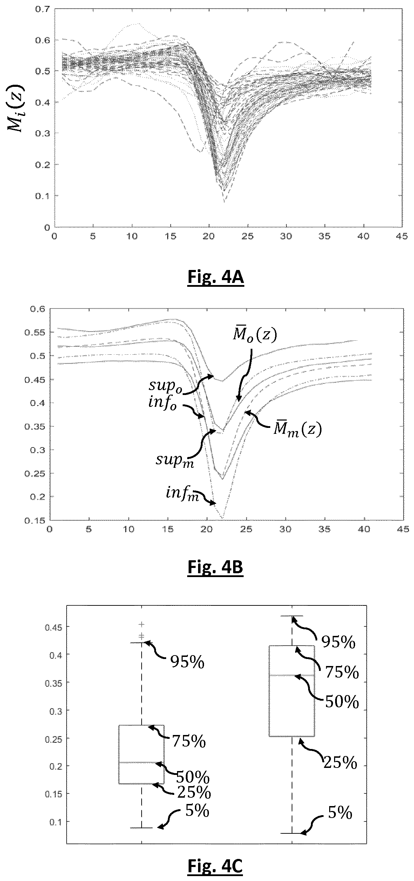

La figure 4A représente des profils du module d'images complexes reconstruites à différentes distances du plan de détection formé par le capteur d'image, chaque profil passant par le centre des régions d'intérêt, telles que représentées sur la figure 3D, correspondant soit à des microorganismes, soit à des particules n'étant pas des microorganismes, par exemple des particules alimentaires. Chacun de ces profils représente une évolution, en fonction de la distance par rapport au plan de détection, du module d'une expression complexe de l'onde d'exposition, à laquelle est exposé le capteur d'image.

La figure 4B représente respectivement, pour des particules correspondant et ne correspondant pas à des microorganismes, une plage de dispersion des profils tels que décrits en lien avec la figure 4A.

La figure 4C montre respectivement, pour des particules correspondant et ne correspondant pas à des microorganismes, une plage de variation de la valeur minimale du profil du module.

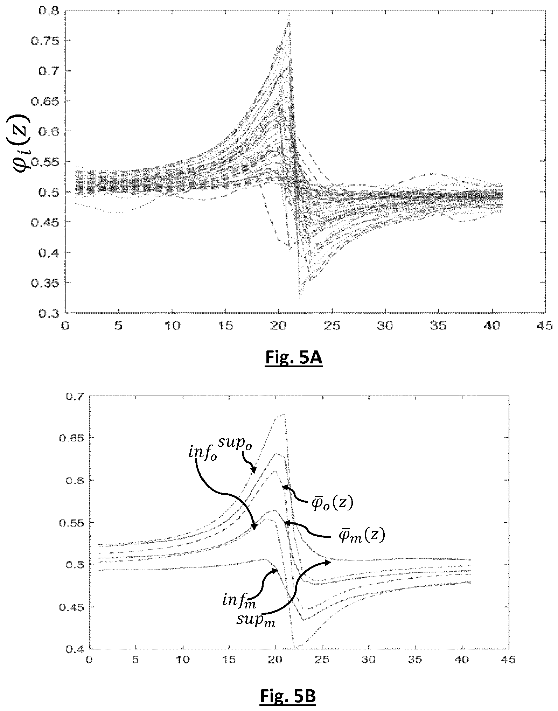

La figure 5A représente des profils de phase d'images complexes reconstruites à différentes distances du plan de détection formé par le capteur d'image, chaque profil passant par le centre des régions d'intérêt, telles que représentées sur la figure 3D, correspondant soit à des microorganismes, soit à des particules n'étant pas des microorganismes, par exemple des particules dites alimentaires. Chacun de ces profils représente une évolution, en fonction de la distance par rapport au plan de détection, de la phase d'une expression complexe de l'onde d'exposition, à laquelle est exposé le capteur d'image. La figure 5B représente respectivement, pour des particules correspondant et ne correspondant pas à des microorganismes, une plage de dispersion des profils tels que décrits en lien avec la figure 5A.

## EXPOSE DE MODES DE REALISATION PARTICULIERS

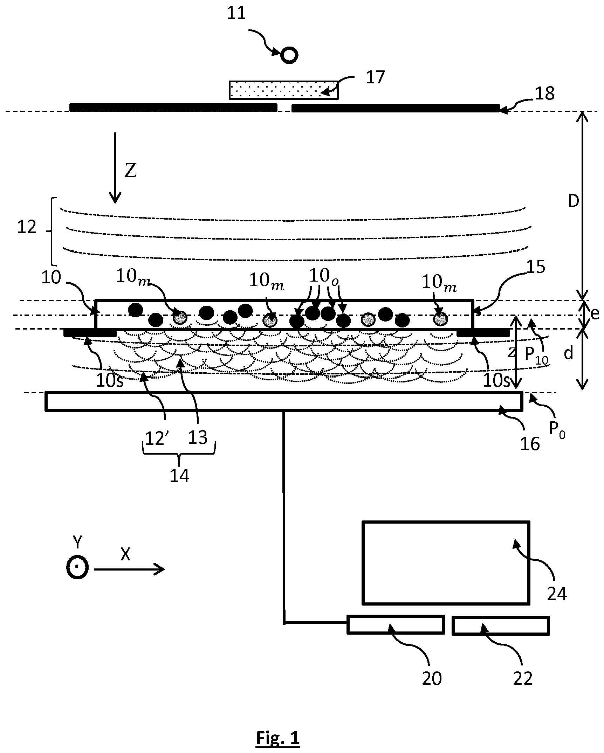

La figure 1 représente un exemple de dispositif selon l'invention. Une source de lumière 11 est apte à émettre une onde lumineuse 12, dite onde lumineuse incidente, se propageant en direction d'un échantillon 10, selon un axe de propagation Z. L'onde lumineuse est émise selon une bande spectrale d'illumination $\Delta\lambda$.

L'échantillon 10 est un échantillon que l'on souhaite caractériser. Selon cet exemple, l'échantillon a été prélevé à partir d'un mélange, destiné à la confection d'un aliment pour animal. Le mélange comporte des aliments, par exemple de la matière grasse, de la viande, et des fibres végétales, en particulier sous forme de farine. Le mélange peut également comporter des compléments alimentaires, par exemple des vitamines. Le mélange comporte également des microorganismes, en particulier des bactéries ou levures, utilisées en tant que complément alimentaire, sous forme de probiotiques. Dans cet exemple, le mélange se présente sous la forme d'une pastille solide sèche, communément désigné par le terme "croquette", destinée à être vendue à destination d'un animal domestique. L'échantillon 10 se présente sous la forme d'une poudre, obtenue après broyage du mélange. La poudre comporte différentes particules, correspondant :

- soit à un aliment ou à un complément alimentaire, désignées par le terme "particules alimentaires" $10_o$;
- soit à des microorganismes $10_m$.

Un objectif de l'invention est d'évaluer une quantité de microorganismes dans l'échantillon. Le terme quantité désigne un nombre ou une concentration. Par microorganisme, on entend notamment une levure, une bactérie, une spore, un champignon ou une cellule, qu'il s'agisse d'une cellule eucaryote ou procaryote, ou une microalgue.

L'échantillon 10 est, dans cet exemple, contenu dans une chambre d'analyse 15. La chambre d'analyse 15 est par exemple une chambre de type Gene Frame® d'épaisseur e=250 $\mu$m. L'épaisseur e de l'échantillon 10, selon l'axe de propagation Z, varie typiquement entre 10 $\mu$m et 1 cm, et est de préférence comprise entre 20 $\mu$m et 500 $\mu$m. L'échantillon s'étend selon au moins un plan $P_{10}$, dit plan médian de l'échantillon, perpendiculaire à l'axe de propagation Z. Il est maintenu sur un support 10s à une distance d d'un capteur d'image 16.

La concentration de microorganismes peut varier entre quelques millions à quelques dizaines ou centaines de milliards de levures par gramme.

La distance D entre la source de lumière 11 et la chambre fluidique 15 est de préférence supérieure à 1 cm. Elle est de préférence comprise entre 2 et 30 cm. Avantageusement, la source de lumière, vue par l'échantillon, est considérée comme ponctuelle. Cela signifie que son diamètre (ou sa diagonale) est préférentiellement inférieur au dixième, mieux au centième de la distance entre la chambre fluidique 15 et la source de lumière. Sur la figure 1, la source de lumière est une diode électroluminescente. Elle est généralement associée à diaphragme 18, ou filtre spatial. L'ouverture du diaphragme est typiquement comprise entre 5 $\mu$m et 1 mm, de préférence entre 50 $\mu$m et 500 $\mu$m. Dans cet exemple, le diaphragme est fourni par Thorlabs sous la référence P150S et son diamètre est de 150 $\mu$m. Le diaphragme peut être remplacé par une fibre optique, dont une première extrémité est placée face à la source de lumière 11 et dont une deuxième extrémité est placée en regard de l'échantillon 10. Le dispositif représenté sur la figure 1 comporte également un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par une source de lumière élémentaire 11 selon un cône d'angle a. De préférence, l'angle de diffusion a varie entre 10° et 80°. Alternativement, la source de lumière peut être une source laser, telle une diode laser. Dans ce cas, il n'est pas utile de lui associer un filtre spatial ou un diffuseur.

De préférence, la bande spectrale d'émission $\Delta\lambda$ de l'onde lumineuse incidente 12 a une largeur inférieure à 100 nm. Par largeur de bande spectrale, on entend une largeur à mi-hauteur de ladite bande spectrale.

Selon un mode de réalisation, la source de lumière 11 comporte plusieurs sources de lumière élémentaires, chacune étant apte à émettre une onde lumineuse incidente dans une bande spectrale $\Delta\lambda_k$. De préférence, les bandes spectrales $\Delta\lambda_k$ des différentes sources de lumière élémentaires sont différentes les unes des autres.

L'échantillon 10 est disposé entre la source de lumière 11 et un capteur d'image 16. Ce dernier s'étend de préférence parallèlement, ou sensiblement parallèlement à un plan médian $P_{10}$ de l'échantillon. Le terme sensiblement parallèlement signifie que les deux éléments peuvent ne pas être rigoureusement parallèles, une tolérance angulaire de quelques degrés, inférieure à 20° ou 10° étant admise. Dans cet exemple, l'échantillon s'étend selon un plan radial XY, perpendiculaire à l'axe de propagation Z.

Le capteur d'image 16 est apte à former une image $I_0$ de l'échantillon 10 selon un plan de détection $P_0$. Dans l'exemple représenté, il s'agit d'un capteur d'image comportant une matrice de pixels, de type CCD ou un CMOS. Le plan de détection $P_0$ s'étend de préférence perpendiculairement à l'axe de propagation Z de l'onde lumineuse incidente 12. La distance d entre l'échantillon 10 et la matrice de pixels du capteur d'image 16 est préférentiellement comprise entre 50 $\mu$m et 2 cm, de préférence comprise entre 100 $\mu$m et 2 mm.

On remarque, dans ce mode de réalisation, l'absence d'optique de grossissement ou de formation d'image entre le capteur d'image 16 et l'échantillon 10. Cela n'empêche pas la présence éventuelle de microlentilles de focalisation au niveau de chaque pixel du capteur d'image 16, ces dernières n'ayant pas de fonction de grandissement de l'image acquise par le capteur d'image, leur fonction étant d'optimiser l'efficacité de détection.

Sous l'effet de l'onde lumineuse incidente 12, les particules présentes dans l'échantillon peuvent engendrer une onde diffractée 13, susceptible de produire, au niveau du plan de détection $P_0$, des interférences, en particulier avec une partie de l'onde lumineuse incidente 12' transmise par l'échantillon. Par ailleurs, l'échantillon peut absorber une partie de l'onde lumineuse incidente 12. Ainsi, l'onde lumineuse 14, transmise par l'échantillon, et à laquelle est exposé le capteur d'image 16, désignée par le terme "onde d'exposition", peut comprendre :

- une composante 13 résultant de la diffraction de l'onde lumineuse incidente 12 par chaque particule de l'échantillon ;
- une composante 12' résultant de la transmission de l'onde lumineuse incidente 12 par l'échantillon, une partie de cette dernière pouvant être absorbée dans l'échantillon.

Ces composantes forment des interférences dans le plan de détection. Aussi, l'image acquise par le capteur d'image comporte des figures d'interférences (ou figures de diffraction), dues aux différentes particules de l'échantillon.

Un processeur 20, par exemple un microprocesseur, est apte à traiter chaque image $I_0$ acquise par le capteur d'image 16, selon les étapes décrites ci-après. En particulier, le processeur est un microprocesseur relié à une mémoire programmable 22 dans laquelle est stockée une séquence d'instructions pour effectuer les opérations de traitement d'images et de calculs décrites dans cette description. Le processeur peut être couplé à un écran 24 permettant l'affichage d'images acquises par le capteur d'image 16 ou calculées par le processeur 20.

Une image $I_0$ acquise par le capteur d'image 16, également appelée hologramme, ne permet pas d'obtenir une représentation suffisamment précise de l'échantillon observé. Comme décrit en lien avec l'art antérieur, on peut appliquer, à chaque image acquise par le capteur d'image, un opérateur de propagation holographique $h$, de façon à calculer une grandeur représentative de l'onde lumineuse d'exposition 14. Il est alors possible de reconstruire une expression complexe $A$ de l'onde lumineuse 14 en tout point de coordonnées ($x,y,z$)

de l'espace, et en particulier dans un plan de reconstruction $P_z$ situé à une distance $|z|$ du capteur d'image 16, dite distance de reconstruction, ce plan de reconstruction étant par exemple le plan $P_{10}$ selon lequel s'étend l'échantillon, avec :

$A(x,y,z) = I_0(x,y,z) * h^*$ désignant l'opérateur produit de convolution.

L'opérateur de propagation $h$ a pour fonction de décrire la propagation de la lumière entre le capteur d'image 16 et un point de coordonnées $(x,y,z)$, situé à une distance $|z|$ du capteur d'image. Il est alors possible de déterminer le module $M(x,y,z)$ et/ou la phase $\varphi(x,y,z)$ l'onde lumineuse 14, à cette distance $|z|$, dite distance de reconstruction, avec :

-   $M(x,y,z) = abs\,[A(x,y,z)]$;
-   $\varphi(x,y,z) = arg\,[A(x,y,z)]$.

Les opérateurs *abs* et *arg* désignent respectivement le module et l'argument.

L'opérateur de propagation est par exemple la fonction de Fresnel-Helmholtz, telle que :

$$h(x,y,z) = \frac{1}{j\lambda z}\, e^{j2\pi\frac{z}{\lambda}} \exp\left(j\pi\frac{x^2+y^2}{\lambda z}\right).$$

Autrement dit, l'expression complexe A de l'onde lumineuse 14, en tout point de coordonnées $(x,y,z)$ de l'espace, est telle que : $A(x,y,z) = M(x,y,z)e^{j\varphi(x,y,z)}$.

Dans la suite de cette description, les coordonnées $(x,y)$ désignent une position radiale dans un plan radial XY perpendiculaire à l'axe de propagation Z. La coordonnée $z$ désigne une coordonnée selon l'axe de propagation Z.

L'expression complexe $A$ est une grandeur complexe dont l'argument et le module sont respectivement représentatifs de la phase et de l'intensité de l'onde lumineuse 14 d'exposition détectée par le capteur d'image 16. Le produit de convolution de l'image $I_0$ par l'opérateur de propagation $h$ permet d'obtenir une image complexe $A_z$ représentant une distribution spatiale de l'expression complexe $A$ dans un plan de reconstruction $P_z$, s'étendant à une distance $|z|$ du plan de détection $P_0$. Dans cet exemple, le plan de détection $P_0$ a pour équation $z = 0$. L'image complexe $A_z$ correspond à une image complexe de l'échantillon dans le plan de reconstruction $P_z$. Elle représente également une distribution spatiale bidimensionnelle de l'expression complexe de l'onde d'exposition 14. Un tel procédé, désigné par le terme reconstruction holographique, permet notamment de reconstruire une image du module ou de la phase de l'onde lumineuse d'exposition 14 dans le plan de reconstruction. Pour cela, on peut former des images $M_z$ et $\varphi_z$ représentant respectivement le module ou la phase de l'image complexe $A_z$, avec $M_z = \mathrm{mod}\,(A_z)$ et $\varphi_z = \arg(A_z)$.

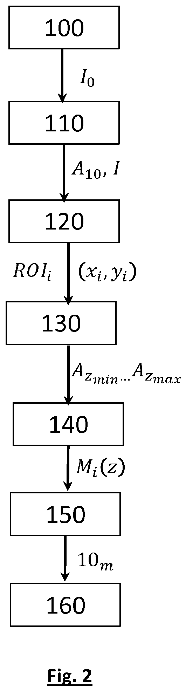

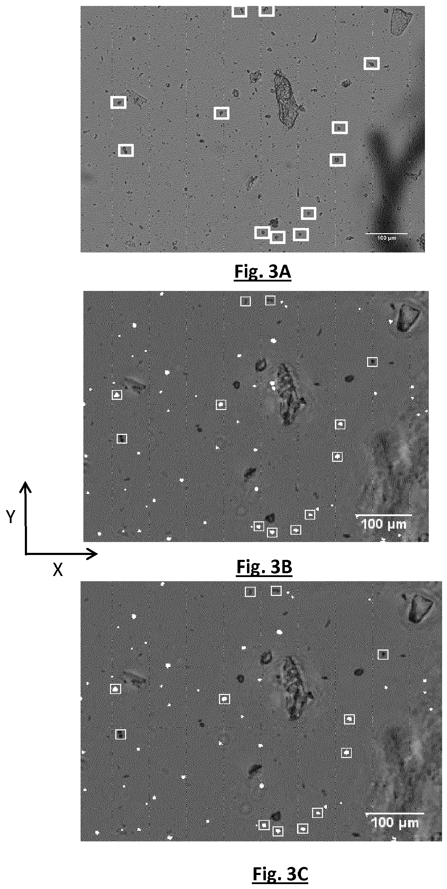

Les inventeurs ont mis au point un procédé de détection de microorganismes, par exemple des levures, ce procédé étant décrit en lien avec la figure 2, et dont certains résultats sont illustrés sur les figures 3B à 3E, 4A à 4C, 5A et 5B. Ces résultats ont été obtenus selon les conditions expérimentales suivantes :

-   Echantillon 10 : il est obtenu par broyage d'une croquette destinée à l'alimentation animale. Il comporte des levures de type *Saccharomyces cerevisiae.* La concentration est d'environ $3\ 10^{10}$ levures par gramme.
-   Source de lumière 11 : diode électroluminescente Cree MC-E Color, comportant trois diodes électroluminescentes pouvant être simultanément ou successivement activées, chaque diode émettant respectivement dans les bandes spectrales $\Delta\lambda$ suivantes : 450nm -465 nm ; 520nm - 535 nm ; 620nm - 630 nm. Dans les essais qui suivent, seule la bande spectrale 620nm - 630 nm a été mise en oeuvre.
-   Capteur d'image 16 : Capteur 8 bits CMOS monochrome 3884 x 2764 pixels, chaque pixel mesurant $1.67\ \mu m$ de côté, la surface de détection s'étendant sur environ 30 mm$^2$. Compte tenu de l'épaisseur de la chambre fluidique, le volume d'échantillon adressé par chaque image s'élève à 7.5 $\mu m^3$.
-   Distance D entre la source de lumière 11 et l'échantillon 10 : 5 cm.
-   Distance d entre l'échantillon 10 et le capteur d'image 16 : 1000 $\mu m$.
-   Epaisseur e de la chambre fluidique 15 : 250 $\mu m$.
-   Diamètre de l'ouverture du filtre spatial 18 : 150 $\mu m$.

Les principales étapes du procédé d'identification et de numération des microorganismes présents dans l'échantillon sont :

-   L'acquisition, par le capteur d'image, d'une image $I_0$ de l'échantillon dans une ou plusieurs bandes spectrales d'illumination.
-   A partir de l'image acquise, l'application d'un opérateur holographique pour obtenir une image complexe représentative de l'onde lumineuse d'exposition 14, dans un plan de reconstruction.
-   La formation d'une image d'observation à partir de l'image complexe et sélection, sur l'image d'observation, de régions d'intérêt $ROI_i$ correspondant à des particules, dites particules d'intérêt $10_i$, chaque particule d'intérêt étant susceptible de corresponde à un microorganisme $10_m$. Cette sélection est effectuée en prenant en compte au moins un critère, par exemple un critère morphologique, et en appliquant le critère à l'image d'observation.
-   La détermination de coordonnées radiales $(x_i,y_i)$, de chaque région d'intérêt $ROI_i$ ainsi sélectionnée, le terme "radial" signifiant dans un plan parallèle au plan de détection.
-   A partir de l'image acquise $I_0$, le calcul d'une grandeur caractéristique, par exemple le module ou la phase, de l'onde lumineuse d'exposition 14, à différentes distances de l'échantillon, dites distances de reconstruction ; cela peut notamment passer par l'obtention d'une pile d'images complexes représentant l'onde lumineuse d'exposition 14, les images complexes s'étendant selon des plans parallèles disposés entre le plan de détection $P_0$ et le plan de l'échantillon $P_{10}$.

- Pour chaque coordonnée radiale correspondant à une région d'intérêt, le calcul d'un profil représentant une évolution de la grandeur caractéristique en fonction de la distance de reconstruction, chaque profil étant associé à une particule d'intérêt.
- A partir des profils respectivement associés à chaque particule d'intérêt, l'identification des particules d'intérêt correspondant à un microorganisme et des particules d'intérêt ne correspondant pas à un microorganisme.

Etape 100: Acquisition d'une image $I_0$ de l'échantillon 10 par le capteur d'image 16, cette image formant un hologramme. Un des intérêts de la configuration sans lentille, représentée sur la figure 1, est le large champ observé, permettant d'adresser simultanément un volume d'échantillon élevé. Cela permet d'observer simultanément plusieurs microorganismes, et ainsi d'obtenir une caractérisation rapide de l'échantillon. Le champ observé dépend de la taille du capteur d'image, en étant légèrement inférieur à la surface de détection de ce dernier, du fait de l'espacement entre les pixels du capteur et l'échantillon. Le champ observé est généralement supérieur à 10 mm$^2$, et est typiquement compris entre 10 mm$^2$ et 50 mm$^2$, ce qui est significativement plus élevé qu'avec un microscope.

Etape 110 : Formation d'une image d'observation.

Du fait de l'absence d'optique de formation d'image, l'image acquise $I_0$ peut comporter un grand nombre de figures d'interférence, et peut ne pas être aisément exploitable pour identifier les particules présentes dans l'échantillon. Les particules sont plus facilement identifiables à partir d'une image complexe reconstruite, dans un plan de reconstruction, par application d'un opérateur de propagation holographique à l'image acquise. Dans cet exemple, cette image complexe est notée $A_{10}$. Elle est obtenue en effectuant une reconstruction holographique dans un plan de reconstruction à une image obtenue à partir de l'image acquise $I_0$. Le plan de reconstruction est avantageusement perpendiculaire à l'axe de propagation Z, et/ou parallèle au plan de détection $P_0$. Il s'agit de préférence d'un plan $P_{10}$ selon lequel s'étend l'échantillon 10, par exemple un plan médian dans lequel s'étendent la majorité des particules.

L'image complexe reconstruite $A_{10}$ est une image complexe comportant des informations de phase et d'amplitude de l'onde lumineuse 14 à laquelle est exposé le capteur d'image 16. En effet, c'est généralement dans ce plan que la résolution spatiale d'une image complexe reconstruite est la meilleure, un tel principe étant à la base des algorithmes dits de focalisation numérique. La coordonnée $z$ du plan de de l'échantillon $P_{10}$ est déterminée soit a priori, notamment lorsque la position de l'échantillon 10 est maîtrisée par rapport au capteur d'image 16, soit par le biais d'une focalisation numérique. La focalisation numérique permet de définir un plan de focalisation $P_{focus}$ en reconstruisant plusieurs images complexes, à différentes distances, et en définissant un critère de netteté de chaque image reconstruite. Le plan de focalisation $P_{focus}$ correspond à celui dans lequel l'image reconstruite présente un critère de netteté optimal. Il correspond au plan médian de l'échantillon $P_{10}$, dans lequel s'étend une majorité des microorganismes. L'image complexe $A_{10}$ est alors formée dans un plan de reconstruction $P_{10}$ correspondant au plan de focalisation $P_{focus}$.

Pour effectuer la reconstruction, une première solution est d'appliquer l'opérateur de propagation $h$ à l'image acquise $I_0$, ou à la racine carrée de l'image acquise $\sqrt{I_0}$, éventuellement normalisée par la valeur moyenne $\overline{I_0}$ de l'image acquise. Cependant, l'image acquise $I_0$ ne comporte pas d'information relative à la phase de l'onde d'exposition 14. De ce fait, la reconstruction holographique s'effectue sur la base d'une information optique incomplète, basée uniquement sur l'intensité de l'onde lumineuse collectée sur le capteur d'image. Il en résulte l'apparition d'un bruit de reconstruction, affectant l'image complexe reconstruite. L'amélioration de la qualité de la reconstruction holographique a fait l'objet de nombreux développements, en mettant en oeuvre des algorithmes fréquemment dénommés « Phase retrieval », permettant une estimation de la phase de l'onde lumineuse à laquelle le capteur d'image est exposé. Ce type d'algorithme permet de limiter le bruit de reconstruction affectant l'image complexe reconstruite $A_{10}$. Un exemple d'algorithme utilisable est par exemple décrit dans US2012/0218379.

Selon une possibilité, l'échantillon est illuminé successivement ou simultanément dans différentes bandes spectrales $\Delta\lambda_k$, et on acquiert, dans le plan de détection $P_0$, une image $I_0(\Delta\lambda_k)$ représentative de chaque bande spectrale. L'algorithme permet d'obtenir une image complexe $A_{10}(\Delta\lambda_k)$ de l'échantillon 10, dans le plan de reconstruction $P_{10}$, dans chaque bande spectrale $\Delta\lambda_k$. Les images complexes ainsi obtenues peuvent être combinées, par exemple en effectuant une moyenne, en chaque pixel, de leur module et de leur phase, ce qui permet de former l'image complexe $A_{10}$. Alternativement, l'image complexe reconstruite est une image complexe $A_{10}(\Delta\lambda_k)$ dans une bande spectrale $\Delta\lambda_k$. Un tel algorithme a été décrit dans la publication S. N. A. Morel, A. Delon, P. Blandin, T. Bordy, O. Cioni, L. Hervé, C. Fromentin, J. Dinten, and C. Allier, "Wide-Field Lensfree Imaging of Tissue Slides," in Advanced Microscopy Techniques IV;

and Neurophotonics II, E. Beaurepaire, P. So, F. Pavone, and E. Hillman, eds., Vol. 9536 of SPIE Proceedings (Optical Society of America, 2015) ainsi que dans la demande de brevet FR1554811 déposée le 28 mai 2015, et plus précisément dans les étapes itératives 100 à 500 décrites dans cette demande. On a montré que l'utilisation de deux ou trois bandes spectrales différentes permet d'obtenir une bonne qualité de reconstruction.

Une autre possibilité, qui correspond au mode de réalisation préféré, est de reconstruire une image complexe en se basant sur une image acquise de l'échantillon lorsque ce dernier est illuminé dans une seule bande spectrale $\Delta\lambda$. L'image complexe peut être obtenue en utilisant un algorithme itératif tel que décrit dans la demande de brevet FR1652500 déposée le 23 mars 2016, et plus précisément selon les étapes 110 à 160 décrites dans ladite demande de brevet.

Comme précédemment mentionné, l'image complexe reconstruite $A_{10}$ correspond à une distribution bidimensionnelle de l'expression complexe de l'onde lumineuse d'exposition 14. A partir de cette image, on peut former une image d'observation $I$ permettant une observation de l'échantillon. L'image d'observation $I$ est une distribution bidimensionnelle d'une grandeur obtenue à partir de l'image complexe. Il peut par exemple s'agir d'une image $M_{10}$ du module de l'image complexe $A_{10}$ ou une image $\varphi_z$ de la phase de l'image complexe $A_{10}$.

A partir de l'image d'observation, on peut effectuer une sélection de régions d'intérêt $ROI_i$ représentatives de particules d'intérêt $10_i$. Comme précédemment évoqué, une particule d'intérêt $ROI_i$ correspond à une particule d'intérêt $10_i$ susceptible de correspondre à un microorganisme $10_m$.

La figure 3A montre une image de l'échantillon précédemment décrit réalisée au microscope. Sur cette image, on peut observer des particules, se présentant sous la forme de points foncés. Les particules correspondant à un microorganisme sont encadrées par un cadre blanc. Bien que longue et fastidieuse, l'observation de l'échantillon au microscope est précise, et peut être considérée comme une méthode de référence.

La figure 3B représente une image d'observation de l'échantillon, telle que précédemment décrite. L'image d'observation correspond ici au module de l'image de l'expression complexe de l'onde lumineuse d'exposition. Elle permet une représentation satisfaisante de l'échantillon, comparable à l'image réalisée au microscope. L'avantage de l'image d'observation est que le champ observé est plus important, par exemple 20 fois plus important, que l'image réalisée au microscope muni d'un objectif x10. L'image représentée sur la figure 3B correspond à une partie d'une image d'observation, correspondant au champ du microscope.

Etape 120 : sélection de régions d'intérêt sur l'image d'observation.

Cette étape vise à détecter, dans l'image d'observation, des régions d'intérêt $ROI_i$, chaque région d'intérêt correspondant à une particule d'intérêt $10_i$. La détection de chaque région d'intérêt $ROI_i$ est effectuée soit manuellement par un opérateur, soit automatiquement, par une analyse morphologique sur l'image d'observation $I$, en mettant par exemple en oeuvre des algorithmes basés sur une corrélation spatiale avec des formes de régions d'intérêt prédéterminées peuvent également être mis en oeuvre. On prend en compte le fait que chaque micro-organisme $10_m$ peut être associé, dans l'image d'observation, à une région d'intérêt $ROI_i$ de forme prédéterminée, pouvant être aisément détectée. Il peut par exemple s'agir d'une forme circulaire ou ellipsoïdale, ou autre. Pour cela, le procédé peut détecter automatiquement chaque région d'intérêt $ROI_i$ en prenant compte un ou plusieurs critères morphologiques correspondant à un microorganisme. A chaque région d'intérêt $ROI_i$ détectée correspond au moins une particule d'intérêt $10_i$, susceptible de correspondre soit à un microorganisme $10_m$, soit à un autre type de particule $10_o$. L'image de la figure 3B montre une sélection de régions d'intérêt résultant d'une détection automatique de taches dont le diamètre est compris entre 3 $\mu$m et 10 $\mu$m, et présentant une excentricité comprise entre 0.9 et 1, une excentricité de 1 correspondant à une tache circulaire. Ces critères de forme et de dimension correspondent à un premier critère morphologique. Les régions d'intérêt sélectionnées, sur la base de premier critère, apparaissent sur la figure 3B sous la forme de taches claires. Les régions d'intérêt encadrées sont celles définies par la méthode de référence, en lien avec la figure 3A, comme correspondant à un microorganisme. On observe un nombre élevé de faux positifs, ces derniers correspondant aux taches claires non encadrées. On observe également quelques faux négatifs, ces derniers correspondant à des taches sombres encadrées, non détectées par l'analyse morphologique, bien que correspondant à des microorganismes.

Afin de réduire davantage le nombre de faux positifs, on a appliqué, aux régions d'intérêt détectées suite à l'application du premier critère morphologique, un deuxième critère d'analyse d'image, basé, pour chaque région d'intérêt, sur une estimation d'un niveau de bruit à l'extérieur de la région d'intérêt, à la périphérie de cette dernière. Pour cela, autour de chaque région d'intérêt $ROI_i$, on définit un voisinage $V_i$. On estime ensuite l'écart type $\sigma(V_i)$ de l'intensité des pixels dans chaque voisinage $V_i$. L'écart type est supposé être représentatif du niveau de bruit. Une région d'intérêt, détectée suite à l'application du premier critère, est confirmée lorsque le niveau de bruit dans le voisinage de cette dernière, est inférieur un certain seuil. Les régions d'intérêt ne satisfaisant pas au deuxième critère sont rejetées. La figure 3C représente les régions d'intérêt sélectionnées par l'application combinée du premier critère morphologique et du critère de niveau de bruit au voisinage de chaque région d'intérêt. Le nombre de faux positifs est inférieur par rapport au résultat représenté sur la figure 3B.

Afin de réduire davantage le nombre de faux positifs, on a appliqué, aux régions d'intérêt détectées suite à l'application des premier et deuxième critères, un troisième critère d'analyse d'image, basé sur une intensité moyenne (c'est-à-dire le niveau de gris moyen), des pixels de chaque région d'intérêt $ROI_i$. Pour cela, on définit une plage d'acceptation, correspondant aux intensités moyennes de régions d'intérêt correspondant aux microorganismes recherchés. Dans cet exemple, on considère qu'une région d'intérêt peut correspondre à un microorganisme lorsque son intensité moyenne est supérieure à 20, sur une plage de 256 niveaux de gris (8 bits). Les régions d'intérêt ne satisfaisant pas au troisième critère sont rejetées. La figure 3D représente les régions d'intérêt $ROI_i$ sélectionnées par l'application combinée des premier, deuxième et troisième critères décrits ci-dessus. Le nombre de faux positifs est inférieur par rapport au résultat représenté sur la figure 3C.

Pour chaque région d'intérêt $ROI_i$ ainsi sélectionnée, sur l'image d'observation $I$, on détermine une position dans le plan radial XY, c'est-à-dire dans un plan parallèle au plan de détection. La position $(x_i,y_i)$ de chaque région d'intérêt $ROI_i$ peut par exemple être obtenue par le calcul de son centroïde.

L'étape 120 permet une sélection de régions d'intérêt $ROI_i$, sur la base de l'image d'observation $I$, en prenant en compte un ou plusieurs critères. Les étapes suivantes, décrites ci-après, permettent d'affiner la sélection, de façon à identifier, parmi les régions d'intérêt sélectionnées, celles correspondant à un microorganisme.

Etape 130 : construction d'une pile d'images complexes.

Cette étape comporte une reconstruction de plusieurs images complexes en effectuant des reconstructions numériques à différentes distances z du plan de détection $P_0$, et en particulier entre le plan de détection $P_0$ et le plan de l'échantillon $P_{10}$, et/ou de part et d'autre du plan de l'échantillon. Les inventeurs ont estimé qu'il était préférable d'obtenir des images complexes de part et d'autre de l'échantillon, de telle sorte que $z_{min} \le z_{10} \le z_{max}$, avec :

- $z_{min}$ représentant une coordonnée minimale, selon l'axe Z, à laquelle une image complexe est reconstruite ;
- $z_{max}$ représentant une coordonnée maximale, selon l'axe Z, à laquelle une image complexe est reconstruite ;
- $z_{10}$ correspondant à une coordonnée du plan $P_{10}$ de l'échantillon 10.

De préférence, les distances de reconstruction $z_{min}...z_{max}$ sont réparties dans une plage de $\pm$ 500 $\mu$m, ou $\pm$ 1000 $\mu$m, voire $\pm$ 2000 $\mu$m de part et d'autre du plan de l'échantillon $P_{10}$. $z_{min}$ et $z_{max}$ correspondent à des bornes de la pile d'image complexe.

Afin d'obtenir la pile d'images complexes, on peut appliquer un opérateur de propagation holographique $h$ à l'image complexe $A_{10}$, décrite en lien avec l'étape 110. L'opérateur de propagation est appliqué selon différentes distances de reconstruction, de façon à obtenir des images complexes

$$A_z = A_{10} * h_z \text{ avec } z_{min} \le z_{10} \le z_{max}.$$

On obtient ainsi une pile d'images complexes $A_{z_{min}}...A_{z_{max}}$.
Les valeurs $z_{min}$ et $z_{max}$ sont les coordonnées minimales et maximales, selon l'axe Z, entre lesquelles l'image complexe $A_{10}$ est propagée.
De préférence, deux plans de reconstruction adjacents sont espacés les uns des autres selon un maillage fin, compris par exemple entre 5 $\mu$m et 50 $\mu$m, et par exemple 25 $\mu$m.
De façon alternative, les images complexes de la pile d'images complexes peuvent être obtenues par une propagation à partir de l'image $I_0$ acquise lors de l'étape 100, selon différentes distances de propagation $z_{min}....z_{max}$. Les images complexes sont de préférence reconstruites en appliquant un algorithme de reconstruction limitant le bruit de reconstruction, comme précédemment décrit. Par "à partir de", on entend à partir de l'image $I_0$ elle-même ou d'une image obtenue par traitement de l'image $I_0$, par exemple une normalisation ou une prise en compte de la racine carrée de l'image.
A l'issue de l'étape 130, on dispose d'une pile d'images complexes $A_{z_{min}}...A_{z_{max}}$, chaque image complexe étant représentative de l'onde lumineuse d'exposition 14, dans un plan de reconstruction. De préférence, chaque plan de reconstruction s'étend parallèlement au plan de détection $P_0$, et/ou à un plan médian de l'échantillon 10.

Etape 140 : formation d'un profil associé à chaque région d'intérêt.

A partir de chaque image complexe formant la pile d'images complexes, on estime une grandeur caractéristique de l'onde lumineuse d'exposition 14, à chaque position radiale $(x_i,y_i)$ sélectionnée lors de l'étape 120, et à une pluralité de distances z de reconstruction du plan de l'échantillon $P_{10}$, ou du plan de détection $P_0$. On forme ensuite un profil représentant une évolution de la grandeur caractéristique en fonction de z, selon l'axe de propagation Z. La grandeur caractéristique peut notamment être établie à partir du module et de la phase de l'expression complexe $A$ décrivant l'onde lumineuse d'exposition 14, en utilisant les images de la pile d'images complexes résultant de l'étape 130. On aboutit alors à un profil de module $M_i(z)$, ou à un profil de phase $\varphi_i(z)$, de l'onde d'exposition 14, selon l'axe de propagation Z, et cela pour chaque position radiale $(x_i,y_i)$ préalablement sélectionnée. Les figures 4A et 5A représentent respectivement des profils de module et des profils de phase passant par les positions radiales $(x_i, y_i)$ représentées sur la figure 3D. L'axe des ordonnées représente la valeur du profil tandis que l'axe des abscisses représente les coordonnées $z$ selon l'axe Z. Plus précisément, dans ces figures, la coordonnée z représente l'index de chaque image formant la pile d'images. Le plan de focalisation correspond à la coordonnée z = 20. Deux plans de reconstruction adjacents correspondent respectivement à deux coordonnées $z$ successives distantes de 2.5 μm.

Chaque profil est obtenu à partir des images de la pile d'images complexes préalablement établie, en effectuant une interpolation entre les coordonnées de deux images reconstruites selon des plans adjacents. Chaque profil est associé à une région d'intérêt $ROI_i$. Les profils correspondant à des microorganismes $10_m$ sont représentés en tirets, tandis que les profils correspondant à d'autres particules $10_o$, dont les particules alimentaires, sont représentés en pointillés.

Etape 150 : identification des microorganismes.

Les profils formés lors de l'étape 140 constituent des signatures des particules d'intérêt $10_i$ associées aux différentes régions d'intérêt $ROI_i$. La nature des particules de l'échantillon étant déterminée par les mesures réalisées au microscope, les inventeurs ont montré que l'analyse des profils permet une discrimination entre les microorganismes $10_m$ et les autres types de particules $10_o$, comprenant les différents types de particules alimentaires, ou autres types de particules, constituant l'échantillon.

Chaque profil prend une valeur minimale à une coordonnée $z_{min}$ comprise entre 20 et 25. Cette valeur minimale est un critère de classification, dans la mesure où elle permet de distinguer un microorganisme d'une autre particule. Cela apparaît plus nettement sur la figure 4B, qui réprésente, pour chaque type de particule, microorganismes $10_m$ en tirets et autres particules $10_o$ en traits pleins :

- une moyenne $\overline{M}_m(z)$ de chaque profil de microorganismes $10_m$ à chaque coordonnée z;
- une plage de dispersion, délimitée par une courbe supérieure $sup_m$ et une courbe inférieure $inf_m$, correspondant à la moyenne $\overline{M}_m(z) \pm 2\sigma_m(z)$, où $\overline{M}_m(z)$ et $\sigma_m(z)$ correspondent respectivement à la moyenne des profils des modules, mesurés pour des microorganismes $10_m$, et leur écart type à la coordonnée z ;
- une moyenne $\overline{M}_o(z)$ de chaque profil de particules alimentaires $10_o$ à chaque coordonnée $z$ ;
- une délimitation supérieure $sup_o$ et inférieure $inf_o$, correspondant à la moyenne $\overline{M}_o(z) \pm 2\sigma_o(z)$, où $\overline{M}_o(z)$ et $\sigma_o(z)$ correspondent respectivement à la moyenne des profils des modules, mesurés pour des particules alimentaires $10_o$, et leur écart type à la coordonnée z.

La figure 4C représente, respectivement un graphe statistique, connu sous le nom de boîte à moustaches, également connu sous la désignation anglosaxonne "box plot", représentant différents fractiles de la distribution des valeurs minimales des profils respectivement pour les microorganismes $10_m$ et pour les autres particules $10_o$. On peut notamment observer, sur chaque population, les fractiles à 5%, 25% (1er quartile), 50% (médiane), 75% (troisième quartile) et 95%. Le graphe à droite correspond aux microorganismes $10_m$ tandis que le graphe de gauche correspond aux autres particules $10_o$. Les croix marquées sur la figure 4C correspondent à des valeurs aberrantes.

La figure 3E montre le résultat de l'identification de microorganismes résultant de la classification des régions d'intérêt sélectionnées sur la figure 3D, la classification étant réalisée sur la base de la valeur minimale des profils du module. On observe une diminution significative du nombre de faux positifs, par rapport à la figure 3D. Cela montre que l'application de critères sur l'image d'observation peut être utile pour effectuer une première sélection de régions d'intérêt pouvant correspondre à des microorganismes. Le recours à une classification sur la base de profils permet d'identifier plus précisément, parmi les régions sélectionnées, les régions d'intérêt correspondant à des microorganismes.

La figure 5A montre différents profils de phase réalisés en considérant la phase de l'onde lumineuse d'exposition, dans les différents plans de reconstruction. La figure 5B est analogue à la figure 4B, et montre une plage de dispersion de la valeur de profils de phase pour des microorganismes (pointillés) et pour d'autres particules (trait continu). On a également représenté les moyennes $\overline{\varphi}m(z)$ et $\overline{\varphi}_o(z)$ des profils correspondant respectivement aux microorganismes $10_m$ et aux autres particules $10_o$. Les courbes $sup_m$, $sup_o$, $inf_m$ et $inf_o$ sont analogues à celles définies en lien avec la figure 4B, autour des valeurs moyennes $\overline{\varphi}_m(z)$ et $\overline{\varphi}_o(z)$. On observe que l'écart entre la valeur minimale et la valeur maximale de chaque profil peut former un indicateur pertinent pour la séparation des microorganismes et des autres particules.

Le procédé peut comporter une étape 160 de dénombrement des microorganismes identifiés lors de l'étape 150. Il s'agit de déterminer leur quantité, sous la forme d'un nombre ou d'une concentration.

Selon une variante, une optique de formation d'image est disposée entre l'échantillon et le capteur d'image, le capteur d'image étant localisé selon une configuration dite défocalisée, le plan focal objet de l'optique étant décalé du plan selon lequel s'étend l'échantillon selon une distance dite de défocalisation. La distance de défocalisation peut être comprise entre 5 μm et 5 mm, et de préférence entre 10 μm et 2 mm. De la même manière qu'en configuration sans lentille, une telle configuration permet l'obtention d'une image dans laquelle chaque microorganisme apparaît sous la forme d'une figure de diffraction, des interférences se produisant entre l'onde lumineuse émise par la source de lumière et se propageant jusqu'au capteur d'image et une onde de diffraction générée par chaque particule de l'échantillon. Le procédé décrit en lien avec les étapes 100 à 160 est applicable à des images acquises selon une telle configuration. Toutefois, une configuration en imagerie sans lentille est préférée, par le plus grand champ d'observation qu'elle procure.

## Revendications

1. Procédé de détection de microorganismes $(10_m)$ dans un échantillon, l'échantillon comportant également des particules dites alimentaires $(10_o)$, formées à partir d'aliments, le procédé comportant les étapes suivantes :

    a) illumination de l'échantillon à l'aide d'une source de lumière (11), la source de lumière émettant une onde lumineuse incidente (12) se propageant vers l'échantillon (10) selon un axe de propagation (Z) ;
    b) acquisition, à l'aide d'un capteur d'image (16), d'une image $(I_0)$ de l'échantillon (10), formée dans un plan de détection $(P_0)$, l'échantillon étant disposé entre la source de lumière (11) et le capteur d'image (16), l'image acquise étant représentative d'une onde lumineuse (14) d'exposition, à laquelle est exposé le capteur d'image (16) sous l'effet de l'illumination ;

    le procédé étant **caractérisé en ce qu'**il comprend également les étapes suivantes :

    c) obtention d'une image d'observation ($I$) de l'échantillon à partir de l'image acquise lors de l'étape b) et sélection, dans l'image d'observation, de régions d'intérêt $(ROI_i)$, chaque région d'intérêt correspondant à une particule d'intérêt $(10_i)$, susceptible de correspondre à un microorganisme $(10_m)$ ou à une particule alimentaire $(10_o)$;
    d) détermination de positions radiales $(x_i, y_i)$, dans un plan parallèle au plan de détection, des particules d'intérêt $(10_i)$ correspondant aux différentes régions d'intérêt sélectionnées;
    e) à partir de l'image acquise $(I_0)$ lors de l'étape b), application d'un opérateur de propagation ($h$), de façon à calculer au moins une grandeur caractéristique $(M, \varphi)$ de l'onde lumineuse d'exposition (14), à chaque position radiale $(x_i, y_i)$ déterminée lors de l'étape d), et cela à une pluralité de distances ($z$) du plan de détection $(P_0)$;

    f) formation d'un profil $(M_i(z), \varphi_i(z))$, représentant une évolution de la grandeur caractéristique calculée lors de l'étape e) selon un axe parallèle à l'axe de propagation (Z) et passant par chaque position radiale $(x_i, y_i)$ déterminée lors de l'étape d), chaque profil étant associé à une particule d'intérêt $(10_i)$;
    g) en fonction de chaque profil formé lors de l'étape f), identification des particules d'intérêt qui correspondent à un microorganisme $(10_m)$ parmi les particules d'intérêt $(10_i)$ sélectionnées lors de l'étape c), de façon à obtenir une discrimination entre les microorganismes et les autres particules alimentaires.

2. Procédé selon la revendication 1, dans lequel l'étape c) comporte les sous-étapes suivantes :

    ci) à partir de l'image acquise lors de l'étape b), application d'un opérateur de propagation ($h$), de façon à former une image complexe de l'échantillon $(A_{10})$, dans un plan de reconstruction dans lequel s'étend l'échantillon ;
    cii) à partir de l'image complexe formée lors de la sous-étape ci), obtention de l'image d'observation ($I$) de l'échantillon;
    ciii) application d'un critère à l'image d'observation obtenue lors de la sous-étape cii), de façon à sélectionner les régions d'intérêt $(ROI_i)$ correspondant à des particules d'intérêt, représentatives d'un microorganisme ;

3. Procédé selon la revendication 2, dans lequel lors de la sous-étape ciii), le critère appliqué est un critère morphologique, paramétré par une forme et/ou une dimension, de telle sorte que les régions d'intérêt sélectionnées sont celles s'étendant, dans l'image d'observation, selon ladite forme et/ou selon ladite dimension.

4. Procédé selon la revendication 3, dans lequel la sous-étape ciii) comporte, pour chaque région d'intérêt $(ROI_i)$ sélectionnée:

    - une définition d'un seuil de niveau de bruit ;
    - une définition d'un voisinage $(V_i)$ autour de la région d'intérêt;
    - une estimation d'un niveau de bruit $(\sigma(V_i))$ dans ledit voisinage;

    de façon à retenir les régions d'intérêt sélectionnées dans le voisinage desquelles le niveau de bruit est inférieur au seuil de niveau de bruit, les autres régions d'intérêt étant rejetées.

5. Procédé selon la revendication 3 ou la revendication 4, dans lequel la sous-étape ciii) comporte, pour chaque région d'intérêt $(ROI_i)$ sélectionnée:

- une définition d'une plage d'acceptation de niveaux d'intensité ;
- un calcul d'un niveau d'intensité représentatif de chaque région d'intérêt ;

de façon à retenir les régions d'intérêt sélectionnées pour lesquelles le niveau d'intensité est compris dans la plage d'acceptation, les autres régions d'intérêt étant rejetées.

6. Procédé selon l'une quelconque des revendications précédentes, dans lequel l'étape e) comporte une formation d'une pile d'images complexes $(A_{z_{min}}...A_{z_{max}})$, chaque image complexe correspondant à une expression complexe de l'onde lumineuse d'exposition (14), dans un plan de reconstruction, les images complexes de la pile d'images complexes étant respectivement formées dans des plans de reconstruction s'étendant entre le plan de détection $(P_0)$ et le plan de l'échantillon $(P_{10})$, selon lequel s'étend l'échantillon, et/ou de part et d'autre du plan de l'échantillon.

7. Procédé selon la revendication 6, dépendant de la revendication 2, dans lequel la pile d'images complexes comporte des images complexes obtenues en appliquant un opérateur de propagation holographique à l'image complexe de l'échantillon $(A_{10})$, formée lors de l'étape c).

8. Procédé selon l'une quelconque des revendications précédentes, dans lequel l'étape g) comporte une détermination d'au moins un paramètre de chaque profil, l'identification des microorganismes étant effectuée en fonction dudit paramètre.

9. Procédé selon la revendication 8, dans lequel le paramètre est une valeur minimale du profil.

10. Procédé selon l'une quelconque des revendications précédentes, dans lequel lors de l'étape f), la propriété optique est obtenue à partir du module d'une expression complexe représentative de l'onde lumineuse d'exposition (14).

11. Procédé selon l'une quelconque des revendications précédentes, dans lequel les microorganismes sont des levures.

12. Procédé selon l'une quelconque des revendications précédentes, dans lequel l'échantillon est obtenu par broyage d'un aliment comportant des microorganismes.

13. Procédé selon l'une quelconque des revendications précédentes, comportant une étape h) de comptage des microorganismes identifiés lors de l'étape g).

14. Dispositif pour l'identification de microorganismes $(10_m)$ disposés dans un échantillon (10), le dispositif comportant :

- une source de lumière (11) apte à émettre une onde lumineuse incidente (12) se propageant vers l'échantillon (10) ;
- un capteur d'image (16) ;
- un support (10s), configuré pour maintenir l'échantillon (10) entre la source de lumière (11) et le capteur d'image (16) ;

un processeur (20), configuré pour recevoir une image $(I_0)$ de l'échantillon acquise par le capteur d'image (16) et à mettre en oeuvre les étapes c) à g) d'un procédé selon l'une quelconque des revendications 1 à 13.

**Fig. 1**

$$100$$

$$I_0$$

$$110$$

$$A_{10}, I$$

$$120$$

$$ROI_i \quad (x_i, y_i)$$

$$130$$

$$A_{z_{min}...}A_{z_{max}}$$

$$140$$

$$M_i(z)$$

$$150$$

$$10_m$$

$$160$$

**Fig. 2**

Fig. 3A

Fig. 3B

Fig. 3C

**Fig. 3D**

$10_i$

**Fig. 3E**

**Fig. 4A**

**Fig. 4B**

**Fig. 4C**

**Fig. 5A**

**Fig. 5B**

**Europäisches Patentamt**
**European Patent Office**
**Office européen des brevets**

# RAPPORT DE RECHERCHE EUROPEENNE

Numéro de la demande

EP 18 19 6638

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2016/151248 A1 (COMMISSARIAT À L'ÉNERGIE ATOMIQUE ET AUX ÉNERGIES ALTERNATIVES [FR]; H) 29 septembre 2016 (2016-09-29) | 1-3,8-14 | INV. G06K9/00 C12Q1/04 G01N33/02 |
| Y | * Abrégé; pages 1, 7, 9, 14, 15; figures 1, 2B, 4A * | 4-7 | ADD. G01N15/14 G03H1/04 |
| Y | WO 2016/118884 A1 (UNIV CALIFORNIA [US]) 28 juillet 2016 (2016-07-28) * Abrégé; alinéas [0046], [0058] * | 4,5 | |
| Y | WO 2016/097092 A1 (COMMISSARIAT À L ÉNERGIE ATOMIQUE ET AUX ÉNERGIES ALTERNATIVES [FR]; B) 23 juin 2016 (2016-06-23) * Abrégé; figure 3 * | 6,7 | |
| A | US 7 616 320 B2 (JAVIDI BAHRAM [US] ET AL) 10 novembre 2009 (2009-11-10) * Abrégé; colonne 2, ligne 5 - ligne 42 * | 1-13 | |
| A | FR 3 031 180 A1 (COMMISSARIAT ENERGIE ATOMIQUE [FR]; BIOMERIEUX SA [FR]) 1 juillet 2016 (2016-07-01) * Abrégé, page 1, lignes 5-11; figure 4 * | 1-13 | |
| A | US 2014/278136 A1 (SHAMSHEYEVA ALENA [US] ET AL) 18 septembre 2014 (2014-09-18) * Abrégé; alinéas [0054], [0271]; figure 21 * | 1-13 | |

DOMAINES TECHNIQUES RECHERCHES (IPC)

G01N
G06K
C12Q
G03H

Le présent rapport a été établi pour toutes les revendications

| Lieu de la recherche | Date d'achèvement de la recherche | Examinateur |
|---|---|---|
| La Haye | 4 février 2019 | Kramer, Joanne |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..............................................................
& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**

EP 18 19 6638

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-02-2019

| Document brevet cité au rapport de recherche | | Date de publication | Membre(s) de la famille de brevet(s) | | Date de publication |
|---|---|---|---|---|---|
| WO 2016151248 | A1 | 29-09-2016 | CN | 107532989 A | 02-01-2018 |
| | | | EP | 3274689 A1 | 31-01-2018 |
| | | | FR | 3034196 A1 | 30-09-2016 |
| | | | JP | 2018514759 A | 07-06-2018 |
| | | | KR | 20180011762 A | 02-02-2018 |
| | | | US | 2018080760 A1 | 22-03-2018 |
| | | | WO | 2016151248 A1 | 29-09-2016 |
| WO 2016118884 | A1 | 28-07-2016 | US | 2018052425 A1 | 22-02-2018 |
| | | | WO | 2016118884 A1 | 28-07-2016 |
| WO 2016097092 | A1 | 23-06-2016 | CN | 107110762 A | 29-08-2017 |
| | | | EP | 3234550 A1 | 25-10-2017 |
| | | | FR | 3030749 A1 | 24-06-2016 |
| | | | JP | 2018507392 A | 15-03-2018 |
| | | | US | 2017284926 A1 | 05-10-2017 |
| | | | WO | 2016097092 A1 | 23-06-2016 |
| US 7616320 | B2 | 10-11-2009 | AUCUN | | |
| FR 3031180 | A1 | 01-07-2016 | CN | 107110764 A | 29-08-2017 |
| | | | EP | 3241014 A1 | 08-11-2017 |
| | | | FR | 3031180 A1 | 01-07-2016 |
| | | | JP | 2018504594 A | 15-02-2018 |
| | | | US | 2017363533 A1 | 21-12-2017 |
| | | | WO | 2016107995 A1 | 07-07-2016 |
| US 2014278136 | A1 | 18-09-2014 | CA | 2942815 A1 | 18-09-2014 |
| | | | EP | 2971055 A1 | 20-01-2016 |
| | | | EP | 3153588 A2 | 12-04-2017 |
| | | | HK | 1217970 A1 | 27-01-2017 |
| | | | US | 2014278136 A1 | 18-09-2014 |
| | | | US | 2016010138 A1 | 14-01-2016 |
| | | | US | 2017218426 A1 | 03-08-2017 |
| | | | WO | 2014145899 A1 | 18-09-2014 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016151248 A
- WO 2016118884 A
- EP 2872870 A
- WO 2016097092 A
- US 20120218379 A
- FR 1554811
- FR 1652500

**Littérature non-brevet citée dans la description**

- Wide-Field Lensfree Imaging of Tissue Slides. **S. N. A. MOREL ; A. DELON ; P. BLANDIN ; T. BORDY ; O. CIONI ; L. HERVÉ ; C. FROMENTIN ; J. DINTEN ; C. ALLIER.** Advanced Microscopy Techniques IV; and Neurophotonics II. Optical Society of America, 2015, vol. 9536